# EUROPEAN PATENT APPLICATION

(11) **EP 4 509 490 A1**
(43) Date of publication of application: **19.02.2025**
(21) Application number: 23755317.7
(22) Date of filing: 31.07.2023
(51) Int. Cl.: C07C 17/25, C07C 21/18

(54) **METHOD FOR PREPARING 2,3,3,3-TETRAFLUOROPROPENE**

(30) Priority: 30.06.2023 CN 202310794010
(71) Applicant: Quzhou Huanxin Fluoro Material Co., Ltd., Quzhou, Zhejiang 324000 (CN)
(72) Inventor: YING, Yongan, Quzhou, Zhejiang 324000 (CN); YANG, Jianmin, Quzhou, Zhejiang 324000 (CN); LI, Guojie, Quzhou, Zhejiang 324000 (CN); LI, Jie, Quzhou, Zhejiang 324000 (CN); LI, Guiguo, Quzhou, Zhejiang 324000 (CN)
(74) Representative: Sarpi, Maurizio
(86) International application number: PCT/CN2023/110264
(87) International publication number: WO 2025/000630

(57) **Abstract**

The present disclosure related to a method for preparing an organic fluorine compound, in particular to a method for preparing 2,3,3,3-tetrafluoropropene. The method includes the steps of enabling 2,3-dichloro-1,1,1-trifluoropropane as a raw material to react under combined action of a Lewis acid catalyst and a fluoridizer to obtain 2-chloro-1,1,1,2-tetrafluoropropane, finally enabling the obtained2-chloro-1,1,1,2-tetrafluoropropane to react with alkali to dehydrochlorinate and obtain the 2,3,3,3-tetrafluoropropene. The method in the present disclosure has the advantages of mild reaction conditions and high selectivity, and therefore the preparation efficiency of the 2,3,3,3-tetrafluoropropene can be improved greatly in a reaction process. Furthermore, according to the method in the present disclosure, the separation and collection difficulties of products can be further reduced effectively, and the preparation cost of the 2,3,3,3-tetrafluoropropene is reduced.

## Description

### TECHNICAL FIELD

The present disclosure relates to a method for preparing an organic fluorine compound, in particular to a method for preparing 2,3,3,3-tetrafluoropropene.

### BACKGROUND ART

2,3,3,3-tetrafluoropropene (HFO-1234yf) is a novel refrigerant, which has widely been applied to the field of refrigeration for automobiles. Compared with conventional refrigerants, the 2,3,3,3-tetrafluoropropene has the advantages of environmental friendliness, energy saving, safety and the like. Compared with the conventional refrigerant R134a, HFO-1234yf exhibits low levels of ozone depletion potential and global warming potential, and thus meeting the environmental requirements. Furthermore, as HFO-1234yf has excellent thermodynamic properties, automobiles are higher in refrigeration effect and lower in energy consumption level after applying such refrigerant. Therefore, HFO-1234yf has been used as the refrigerant for automobile air conditioners in many countries and regions currently, while the conventional refrigerant R134a is facing a trend of being gradually eliminated.

At present, the 2,3,3,3-tetrafluoropropene has shortcomings in production. For example, consumption of raw materials and energy sources is higher, side reactions are prone to occur to reduce the purity and yield of products, and additives and auxiliaries are required to improve properties of the materials in the reaction process and increase the yield of the products. However, these additives and auxiliaries may increase the production cost and reduce the purity of the products, and raises the risk of environmental pollution.

For example, the patent with a publication number of CN101979364B has disclosed a method for preparing 2,3,3,3-tetrafluoropropene, which includes: step 1, enabling 3,3,3-trifluoropropene CF₃CH=CH₂(I) as a raw material to perform an addition reaction with chlorine under irradiation of an ultraviolet lamp at -10°C-100°C by using a halogenated hydrocarbon as a solvent, to prepare 3,3,3-trifluoro-1,2-dichloropropane CF₃CHClCH₂Cl(II); step 2, enabling the compound of Formula (II) to perform a dehydrochlorination reaction at 0-150°C to by using an alkali metal hydroxide as a catalyst and polymer polyether or macromolecular ether as an assistant catalyst, to produce CF₃CCl=CH₂(III); step 3, enabling the compound of Formula (III) to perform an addition reaction with HF at 5°C-100°C under 0.2-1.0 MPa by using SnCl₄ and TiCl₄ fluorosulfonic acids as catalysts and an aprotic inert compound as an inhibitor, to produce CF₃CClFCH₃(IV); and step 4, enabling the compound of Formula (IV) to perform a dehydrochlorination reaction at 0-100°C under 0-0.5 MPa in the presence of an alkali metal catalyst by using an organic amine halide as a phase transfer catalyst, to produce HFO-1234yf. In this method, a side reaction is prone to occur in the addition reaction process of the 2,3,3,3-trifluoropropylene and the chlorine in step 1, resulting in lower yield and selectivity of the 2,3,3,3-trifluoro-1,2-dichloropropane, and consequently the overall yield of the 2,3,3,3-tetrafluoropropene is lower.

For example, the patent with a publication number of EP2546224B1 has disclosed a method for producing a fluorinated olefin, which specifically includes the steps of fluorating CCl₂=CClCH₂Cl by using a mixture of Cr₂O₃ and FeCl₃/C as a catalyst so as to synthesize a compound CF₃CCl=CH₂, then converting the CF₃CCl=CH₂ into CF₃CFClCH₃ by using SbCl₅ as the catalyst, and finally converting the CF₃CFClCH₃ into HFO-1234yf selectively. However, in this method, high temperature and the catalyst containing heavy metal chromium are required in the implementation process, and thus the process is more complicated, easily leads to safety-related problems, and has serious environmental hazards.

### SUMMARY

In order to overcome the shortcomings of excessive side reactions, uneasy preparation of catalysts, serious environmental hazards, and lower overall yield and selectivity of the method for synthesizing 2,3,3,3-tetrafluoropropene in the prior art, the present disclosure provides a novel method for preparing 2,3,3,3-tetrafluoropropene to overcome the above shortcomings.

In order to achieve the above objective of the present disclosure, the present disclosure provides the following technical solution:

The present disclosure provides a method for preparing 2,3,3,3-tetrafluoropropene, including the following steps:

enabling 2,3-dichloro-1,1,1-trifluoropropane as a raw material to react under combined action of a Lewis acid catalyst and a fluoridizer to obtain 2-chloro-1,1,1,2-tetrafluoropropane, finally enabling the obtained 2-chloro-1,1,1,2-tetrafluoropropane to react with alkali to dehydrochlorinate and obtain the 2,3,3,3-tetrafluoropropene.

In the present disclosure, the 2-chloro-1,1,1,2-tetrafluoropropane (244bb) is firstly prepared from the 2,3-dichloro-1,1,1-trifluoropropane (243db) as the raw material in a liquid phase. In this process, the 2,3-dichloro-1,1,1-trifluoropropane (243db) is rearranged and fluorated under combined action of the Lewis acid and the fluridizer, and the two reactions of rearrangement and fluorination occur simultaneously. The 2,3-dichloro-1,1,1-trifluoropropane undergoes the rearrangement reaction in the presence of the Lewis acid catalyst to form the 2,2-dichloro-1,1,1-trifluoropropane. The 2,2-dichloro-1,1,1-trifluoropropane performs a halogen-exchange fluorination reaction in the presence of the fluoridizer in situ immediately, and finally a chlorine atom on No. 2 carbon atom is exchanged with a fluorine atom to obtain a key intermediate product of 2-chloro-1,1,1,2-tetrafluoropropane (244bb). A relevant reaction process is shown in Formula (I):

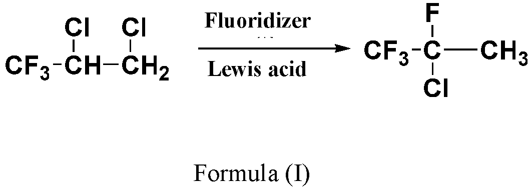

Generally, the 2,3-dichloro-1,1,1-trifluoropropane serves as the raw material. As two chlorine atoms are on No. 2 carbon atom and No. 3 carbon atom, the chlorine atom on No. 3 carbon atom will be substituted by fluorination in preference to obtain 2-chloro-1,1,1,3-tetrafluoropropane. The chemical is a precursor for preparation of 1234ze. For example, the patents EP2634165 and WO2008/54781 have disclosed gas phase fluorination processes of 243db respectively. According to the present disclosure, the process includes the steps of rearranging the 2,3-dichloro-1,1,1-trifluoropropane under action of a polar mixed solvent and a catalyst into 2,2-dichloro-1,1,1-trifluoropropane, and then performing in-situ liquid-phase fluorination in a reactor to obtain 2-chloro-1,1,1,2-tetrafluoropropane. The generation of 2-chloro-1,1,1,3-tetrafluoropropane is avoided.

For the rearrangement step of the 2,3-dichloro-1,1,1-trifluoropropane, the applicant found that it had been mentioned in the patent CN113292392A. This patent has disclosed a rearrangement reaction between saturated hydrochlorofluorocarbons, and a method for preparing fluorinated alcohol using the same. Different from the above patents, the present disclosure emphasizes that the saturated hydrochlorofluorocarbons perform a fluorination reaction immediately after the rearrangement reaction, to obtain saturated hydrochlorofluorocarbons with higher fluorine content, instead of preparing the fluorinated alcohol.

Halogen-exchange fluorination refers to a method for producing a fluorine compound containing fluorine atoms by substituting other halogens with fluorine. In the prior art, a halogen-exchange fluorination method generally includes a halogen-exchange reaction between a compound containing halogen atoms and inorganic halides such as t potassium fluoride at high temperature. However, the conventional halogen-exchange fluorination method has two following shortcomings: (1) the reaction temperature is higher, resulting in a reaction process out of control; and (2) for polyhalogen compounds, all halogen atoms may be exchanged in the halogen exchange process.

In the present disclosure, a liquid phase halogen-exchange fluorination process is selected, the complex of an organic alkali and a hydrogen fluoride reacts with the 2,2-dichloro-1,1,1-trifluoropropane to prepare 2-chloro-1,1,1,2-tetrafluoropropane (244bb). The applicant found that the properties of the solvent have obvious influence on process conditions of the halogen-exchange fluorination reaction. The applicant further found that a certain amount of nitrogen-containing compounds are added therein to serve as a cosolvent, and the nitrogen-containing compounds could form hydrogen bonds with the hydrogen fluoride, thereby regulating the nucleophilicity of fluoride ions. Therefore, the nucleophilicity of the fluoride ions is controlled by selecting nitrogen-containing heterocyclic compounds with different alkalinities to form the hydrogen bonds with the hydrogen fluoride. The applicant found that the most preferred nitrogen-containing compounds are those that are capable of forming strong hydrogen bonds with the hydrogen fluoride and are organic alkalis with high steric hindrance at the same time. In the environment of the aprotic polar solvent and the nitrogen-containing compounds as the cosolvent, the fluoride ions in the fluorination reaction are "bare", leading to a higher nucleophilicity, that is, a binding capacity between a nucleophilic reagent and a carbon atom is improved. The fluoride ions are coperiodic with the carbon atoms, orbit sizes are similar, overlapping is good, and F-C bond energy is high; and therefore, the fluoride ions in the cosolvent are maximal in nucleophilicity, causing the fluorination reaction to occur in a mild process, which obviously improves the selectivity of monofluorinated substituents in the cosolvent.

Finally, in the present disclosure, in the process of preparing the 2,3,3,3-tetrafluoropropylene (1234yf) from the 2-chloro-1,1,1,2-tetrafluoropropane (244bb), it was found from the comparison between the finally obtained product of 2,3,3,3-tetrafluoropropylene (1234yf) and the 2-chloro-1,1,1,2-tetrafluoropropane (244bb) as the raw material used in this step that both of them changed in phases at room temperature and normal pressure. For example, at room temperature and normal pressure, the 2-chloro-1,1,1,2-tetrafluoropropane (244bb) is in a liquid phase, while the final product of 2,3,3,3-tetrafluoropropylene (1234yf) is in a gas phase; and therefore, the 2,3,3,3-tetrafluoropropylene (1234yf) may be separated from the 2-chloro-1,1,1,2-tetrafluoropropane (244bb) upon completion of preparation, which prevents impurities from being introduced into the product. Thus, the collection and purification difficulties in the reaction process can be reduced by the technical means in the present disclosure, thereby benefiting industrial production.

In conclusion, according to the present disclosure, the production efficiency of the 2,3,3,3-tetrafluoropropylene (1234yf) can be improved under the milder reaction conditions by the two reactions. Meanwhile, the separation and collection difficulties of the products are reduced, and impurities are prevented from being introduced into the product.

Preferably, the preparation process of the 2-chloro-1,1,1,2-tetrafluoropropane is implemented in a mixed solvent of the aprotic polar solvent and the cosolvent.

Preferably, the cosolvent is the nitrogen-containing compounds, with an amount added ranging from 5% to 20% of a mass of the aprotic solvent, resulting in the formation of the mixed solvent.

Preferably, the cosolvent is any one, any two or a combination of more of 1,3-dimethyl-3,4,5,6-tetrahydro-2(1H)-pyrimidinone, 1,3-dimethyl-2-imidazolinone, hexamethylphosphamide, imidazole, quinoline and isoquinoline, as well as other substituted or non-substituted nitrogen-containing five-membered ring compounds, and substituted or non-substituted nitrogen-containing six-membered ring compounds.

Preferably, the Lewis acid catalyst is a metal halide and/or a metal oxide, wherein halogen is chlorine or fluorine.

Preferably, the Lewis acid catalyst is any one or a combination of more of aluminum trichloride, ferric trichloride, tin trichloride, boron trifluoride, niobium pentachloride, ferric oxide and zinc oxide.

Preferably, in the rearrangement and selective halogen exchange fluorination processes in (S. 1), the solvent is the aprotic polar solvent.

Further preferably, in the rearrangement process in (S.2), the aprotic polar solvent includes any one or a mixture of more of dimethyl sulfoxide, sulpholane, acetonitrile, N, N-dimethylformamide, nitrobenzene, N-methylpyrrolidone, acetic anhydride and acetone.

Preferably, in the process of preparing the 2-chloro-1,1,1,2-tetrafluoropropane, a reaction temperature ranges from 30°C to 150°C, and reaction time ranges from 12 to 72 hours.

Preferably, the organic alkali in the complex of the organic alkali and the hydrogen fluoride includes any one of trimethylamine, triethylamine, diisopropylethylamine, diisopropylamine, dimethylisopropylamine, N, N-dimethylformamide, pyridine, piperidine, imidazole, N-methylimidazole, morpholine, pyrrolidine and hexamethylphosphoramide.

Correspondingly, further preferably, the complex of the organic alkali and the hydrogen fluoride is any one of a complex of trimethylamine and a hydrogen fluoride, a complex of triethylamine and a hydrogen fluoride, a complex of diisopropylethylamine and a hydrogen fluoride, a complex of diisopropylamine and a hydrogen fluoride, a complex of dimethylisopropylamine and a hydrogen fluoride, a complex of N,N-dimethylformamide and a hydrogen fluoride, a complex of pyridine and a hydrogen fluoride, a complex of piperidine and a hydrogen fluoride, a complex of imidazole and a hydrogen fluoride, a complex of N-methylimidazole and a hydrogen fluoride, a complex of morpholine and a hydrogen fluoride, a complex of pyrrolidine and a hydrogen fluoride, and a complex of hexamethylphosphoramide and a hydrogen fluoride.

Preferably, in the dehydrochlorination process, the alkali includes any one of sodium hydroxide, potassium hydroxide, sodium methoxide, sodium ethoxide, potassium tert-butoxide, and potassium tert-butoxide.

Preferably, in the dehydrochlorination process, a reaction temperature ranges from 35°C to 90°C, the 2,3,3,3-tetrafluoropropylene is condensed and liquefied after being introduced into a cold trap, thereby facilitating collection of the 2,3,3,3 -tetrafluoropropyl ene.

Therefore, compared with the prior art, the present disclosure has the following beneficial effects:

The method in the present disclosure has the advantages of mild reaction conditions and high selectivity, and therefore the preparation efficiency of the 2,3,3,3-tetrafluoropropene can be improved greatly in the reaction process. Furthermore, according to the method in the present disclosure, the separation and collection difficulties of products can be further reduced effectively, and the preparation cost of the 2,3,3,3-tetrafluoropropene is reduced.

### DETAILED DESCRIPTION OF THE EMBODIMENTS

The present disclosure will be further described with reference to the embodiments. A person having ordinary skilled in the art can implement the present disclosure based on these descriptions. Furthermore, the embodiments of the present disclosure involved in the following description are merely a part of embodiments of the present disclosure, and not all of the embodiments. Therefore, all other embodiments obtained by a person having ordinary skill in the art based on the embodiments in the present disclosure without involving inventive effort should fall within the protection scope of the present disclosure.

2,3-dichloro-1,1,1-trifluoropropane used in the embodiment is commercially available. Part of methods for preparing the 2,3-dichloro-1,1,1-trifluoropropane have been disclosed in the prior art, specifically shown as follows.

A method 1 for preparing 2,3-dichloro-1,1,1-trifluoropropane includes the following steps:

carbon tetrachloride (CCl₄) as a cheap raw material and ethylene are synthesized, that is, the ethylene reacts with the carbon tetrachloride to produce 1,1,1,4-tetrachloropropane, the 1,1,1,4-tetrachloropropane reacts with HF to produce 3-chloro-1,1,1-trifluoropropane, HCl is removed from the 3-chloro-1,1,1-trifluoropropane under action of the alkali (such as KOH) to obtain 3,3,3-trifluoropropene, and 2,3-dichloro-1,1,1-trifluoropropane is obtained through an addition reaction between the 3,3,3-trifluoropropene and Cl₂.

A method 2 for preparing 2,3-dichloro-1,1,1-trifluoropropane as a raw material includes the following steps: 3,3,3-trifluoropropene serves as the raw material, the 3,3,3-trifluoropropene and chlorine are preheated to 100°C-150°C respectively, and enter a reactor with a catalyst for reaction after being mixed by a mixer, wherein a molar ratio of the 3,3,3-trifluoropropene to the chlorine is 0.5: 1 to 2:1, a reaction temperature ranges from 100°C to 150°C, and a reaction pressure ranges from 0 to 1.0 MP.

It should be noted that the above-mentioned methods for preparing 2,3-dichloro-1,1,1-trifluoropropane are for reference only, and may not fall within the required protection scope of the claims.

### Embodiment 1

(S.1) At normal temperature, 8.3 g of 2,3-dichloro-1,1,1-trifluoropropane is introduced into a mixed solution of 50 ml of dimethyl sulfoxide and 5 g of 1,3-dimethyl-3,4,5,6-tetrahydro-2(1H)-pyrimidinone to be injected into a reactor, then 1 g of aluminum trichloride and a complex of triethylamine and a hydrogen fluoride Et₃N·3HF (17ml) are added to the reactor, stirred to react for 2 hours, and then heated to 90°C with stirring to continue reacting for 24 hours, and a reaction mixture is poured into ice water (500 ml) upon completion of the reaction, and neutralized with a concentrated ammonia solution. An organic phase is separated, and dried by MgSO₄. After distillation and purification, it is confirmed by gas chromatography to obtain 6.72 g of 2-chloro-1,1,1,2-tetrafluoropropane (244bb) (yield: 89.7%).

(S.2) Under nitrogen protection, 50 ml of potassium tert-butoxide (6.8 g) tetrahydrofuran solution is added to the reactor, the reactor is cooled to -5°C, the 2-chloro-1,1,1,2-tetrafluoropropane(7.Sg) is added to the reactor, and then the mixture is slowly heated to 65°C with stirring for reaction to produce a large amount of gas; the gas is introduced into a cold trap with dry ice as a refrigerant, stirred to react for 4 hours, and then the reaction is stopped; and products in the cold trap are collected, and confirmed by the gas chromatography to obtain 5.49 g of 2,3,3,3-tetrafluoropropylene (HFO-1234yf) (yield: 96.3%).

### Embodiment 2

(S.1) At normal temperature, 8.3 g of 2,3-dichloro-1,1,1-trifluoropropane is introduced into a mixed solution of 50 ml of dimethyl sulfoxide and 2.5g of 1,3-dimethyl-2-imidazolone to be added to a nickel-iron alloy reactor, then 1 g of aluminum chloride and Et₃N·3HF(17ml) are added to the reactor, heated to 50°C with stirring to react for 5 hours, and then heated to 80°C to continue reacting for 12 hours, and a reaction mixture is poured into ice water (500 ml) upon completion of the reaction, and neutralized with a concentrated ammonia solution. An organic phase is separated, and dried by MgSO₄. After distillation and purification, it is confirmed by gas chromatography to obtain 6.29g of 2-chloro-1,1,1,2-tetrafluoropropane (244bb) (yield: 83.9%).

(S.2) is shown in Embodiment 1.

### Embodiment 3

(S.1) At normal temperature, 1 g of aluminum chloride, 8.3 g of 2,3-dichloro-3,3,3-trifluoropropane and 50 ml of dimethyl sulfoxide are added to a nickel-iron alloy reactor, and heated to 100°C with stirring to react for 36 hours; the reactor is cooled to 20°C, and 10 g of hexamethylphosphamide, and Et₃N·3HF (17 ml) are added to the reactor again, and heated to 80°C with stirring to react for 12 hours; and a reaction mixture is poured into ice water (500 ml) upon completion of reaction, and neutralized with a concentrated ammonia solution. An organic phase is separated, and dried by MgSO₄. After distillation and purification, it is confirmed by gas chromatography to obtain 4.32g of 2-chloro-1,1,1,2-tetrafluoropropane (244bb) (yield: 57.7%).

(S.2) is shown in Embodiment 1.

### Comparative Example 1

Comparative Example 1 is different from Embodiment 1 in that the addition of 1,3-dimethyl-3,4,5,6-tetrahydro-2(1H)-pyrimidinone in (S.1) is canceled, and 2.06 g of 2-chloro-1,1,1,2-tetrafluoropropane (244bb) (yield: 27.6%) is obtained through separation finally.

### Comparative Example 2

(S.1) 8.3 g of 2,3-dichloro-1,1,1-trifluoropropane is introduced into a mixed solution of 50 ml of dimethyl sulfoxide and 25 g of 1,3-dimethyl-3,4,5,6-tetrahydro-2(1H)-pyrimidinone to be added to a reactor; then, 1 g of aluminum chloride and Et₃N·3HF (17 ml) are added to the reactor, and heated to 80°C with stirring to react for 24 hours; and a reaction mixture is poured into ice water (500 ml) upon completion of the reaction, and neutralized with a concentrated ammonia solution. An organic phase is separated, and dried by MgSO₄. After distillation and purification, it is confirmed by gas chromatography to obtain 4.79g of 2-chloro-1,1,1,2-tetrafluoropropane (244bb) (yield: 63.9%).

It can be known from Embodiments 1 to 3 that the 2,3,3,3-tetrafluoropropene can be prepared from the 3,3,3-trifluoropropene as the raw material under milder reaction conditions by the preparation method in the present disclosure. Compared with the prior art, the method in the present disclosure has the effects of mild reaction conditions, high selectivity and low cost of reaction raw materials. Therefore, the preparation efficiency of the 2,3,3,3-tetrafluoropropene can be improved in the reaction process, meanwhile the separation and collection difficulties of the products are reduced effectively, and the manufacturing cost of the 2,3,3,3-tetrafluoropropene is reduced.

Through comparison of Embodiment 1 with Comparative Example 1, it can be known that, as the nitrogen-containing heterocyclic compounds as the cosolvent are not added in (S. 1) in Comparative Example 1, the nucleophilicity of fluoride ions in a reaction system is reduced greatly, resulting in an obvious decrease in the yield of 2-chloro-1,1,1,2-tetrafluoropropane. However, it can be known from Comparative Example 2 that the addition of the excessive nitrogen-containing heterocyclic compounds as the cosolvent may result in overhigh nucleophilicity of the fluoride ions, leading to an obvious increase in difluorinated substituents, and the yield of the final target product of 2-chloro-1,1,1,2-tetrafluoropropane is reduced significantly.

The specific embodiments described herein are only used for illustrating the spirit of the present disclosure. A person skilled in the art to which the present disclosure pertains may make various modifications or supplementation or replacement in a similar manner to the described specific embodiments, which should not depart from the spirit of the present disclosure or exceed the scope defined by the appended claims.

## Claims

1. A method for preparing 2,3,3,3-tetrafluoropropene, **characterized by** comprising the following steps:
enabling 2,3-dichloro-1,1,1-trifluoropropane as a raw material to react under combined action of a Lewis acid catalyst and a fluoridizer to obtain 2-chloro-1,1,1,2-tetrafluoropropane, finally enabling the obtained2-chloro-1,1,1,2-tetrafluoropropane to react with alkali to dehydrochlorinate and obtain the 2,3,3,3-tetrafluoropropene.

2. The method for preparing the 2,3,3,3-tetrafluoropropene according to claim 1, **characterized in that**
the Lewis acid catalyst is a metal halide and/or a metal oxide, wherein halogen is chlorine or fluorine.

3. The method for preparing the 2,3,3,3-tetrafluoropropene according to claim 2, **characterized in that**
the Lewis acid catalyst is any one or a combination of more of aluminum trichloride, ferric trichloride, tin trichloride, boron trifluoride, niobium pentachloride, ferric oxide and zinc oxide.

4. The method for preparing the 2,3,3,3-tetrafluoropropene according to claim 1, **characterized in that**
in the process of preparing the 2-chloro-1,1,1,2-tetrafluoropropane, a reaction temperature ranges from 30°C to 150°C, and reaction time ranges from 12 to 72 hours.

5. The method for preparing the 2,3,3,3-tetrafluoropropene according to any one of claims 1-4, **characterized in that**
the fluoridizer is a complex of an organic alkali and a hydrogen fluoride.

6. The method for preparing the 2,3,3,3-tetrafluoropropene according to claim 5, **characterized in that**
the organic alkali in the complex of the organic alkali and the hydrogen fluoride comprises any one of trimethylamine, triethylamine, diisopropylethylamine, diisopropylamine, dimethylisopropylamine, N, N-dimethylformamide, pyridine, piperidine, imidazole, N-methylimidazole, morpholine, pyrrolidine and hexamethylphosphoramide.

7. The method for preparing the 2,3,3,3-tetrafluoropropene according to any one of claims 1-4, **characterized in that**
the process of preparing the 2-chloro-1,1,1,2-tetrafluoropropane is performed in a liquid phase.

8. The method for preparing the 2,3,3,3-tetrafluoropropene according to claim 7, **characterized in that**
the process of preparing the 2-chloro-1,1,1,2-tetrafluoropropane is performed in a mixed solvent of an aprotic polar solvent and a cosolvent.

9. The method for preparing the 2,3,3,3-tetrafluoropropene according to claim 8, **characterized in that**
the cosolvent is nitrogen-containing compounds with an amount added ranging from 5% to 20% of a mass of the aprotic solvent, resulting in a formation of the mixed solvent.

10. The method for preparing the 2,3,3,3-tetrafluoropropene according to claim 8, **characterized in that**
the aprotic polar solvent comprises any one or a mixture of more of dimethyl sulfoxide, sulpholane, acetonitrile, N, N-dimethylformamide, nitrobenzene, N-methylpyrrolidone, acetic anhydride and acetone; and
the cosolvent is any one, any two or a combination of more of 1,3-dimethyl-3,4,5,6-tetrahydro-2(1H)-pyrimidinone, 1,3-dimethyl-2-imidazolinone, hexamethylphosphamide, imidazole, quinoline and isoquinoline, as well as other substituted or non-substituted nitrogen-containing five-membered ring compounds, or substituted or non-substituted nitrogen-containing six-membered ring compounds.
